# EUROPEAN PATENT SPECIFICATION

(11) **EP 3 202 347 B1**
(45) Date of publication and mention of the grant of the patent: **22.05.2019**
(21) Application number: 17154521.3
(22) Date of filing: 03.02.2017
(51) Int. Cl.: A61B 17/72

(54) **INTRAMEDULLARY BONE DISTRACTION IMPLANT**
INTRAMEDULLÄRES IMPLANTAT ZUR KNOCHENDISTRAKTION
IMPLANT INTRAMÉDULLAIRE DE DISTRACTION OSSEUSE

(30) Priority: 03.02.2016 TR 201601411
(43) Date of publication of application: 09.08.2017
(73) Proprietor: Yeditepe Universitesi, 34755 Istanbul (TR); Arslan Makina Dokum Sanayi ve Ticaret Limitd Sirketi, 34570 Istanbul (TR)
(72) Inventor: OKYAR, A. Fethi, ISTANBUL (TR); SAFAK, Koray K., ISTANBUL (TR); SIMSEK, Mustafa, ISTANBUL (TR); EGRICAN, A. Nilufer, ISTANBUL (TR)
(74) Representative: Dericioglu Kurt, Ekin

(56) References cited:
- WO-A1-03/068089
- WO-A1-2010/134078
- FR-A1- 2 847 153

## Description

### Field of the Invention

The present invention relates to a moving intramedullary bone distraction implant, which is placed into the intramedullary canal in cases of uncontrolled bone growth or insufficient new bone formation occurring in the patient's body, and which can be extended or shortened.

### Background of the Invention

Limb length difference in human body is a functional orthopedic problem beyond a cosmetic problem. Today, treatment methods for limb length discrepancy have an important role in pathology treatment due to both the walking pattern changes associated with balance disorder in the frontal plane and degenerative disorders occurring in the axial skeleton.

Today, a comfortable postoperative follow up as well as a short hospitalization period are desired for limb lengthening as for all treatments. Treatment of the lower limb length differences has been a controversial issue for many years in the world of orthopedics, and it has been an issue which, at times, is not leaned towards by the surgeons due to the possible complications thereof. Various periods wherein different treatments have been prominent in the history of orthopedics have followed one another. One of these treatment methods is the telescopic intramedullary nail system which enables lengthening in the bone. The telescopic intramedullary nail systems include a rod-shaped mechanism placed into the bone and a mobile part which moves by means of the screw system in the rod form and enables lengthening of the bone. In the system that is formed, only forward movement is applied for lengthening the bone; and a shortening operation cannot be carried out on the lengthening apparatus in cases of uncontrollable growth that theoretically may be guided by the patient or due to insufficient new bone formation.

A motor is used to provide the driving force for the moving part. However, the problem of length control related to intermittent start and stopping of the motor was reported. In the state of the art, motor nail systems, which are powered by the magnet biased antennas in order to control the motor drive, are applied. However, the applied magnet biased power antennas cause problems in terms of patient comfort and reliability, and thus there is a need of seeking alternative solutions.

Document FR2847153 relates to devices which are used to move two bone portions in relation to one another. The inventive device is essentially characterised in that it consists of: a sleeve, a tube which is mounted to slide in translation inside the afore mentioned sleeve, a controllable motor which is disposed inside the sleeve, a nut portion and means of connecting the nut portion to the sliding tube. According to the invention, the output shaft of the reducer comprises a threaded part which is mounted to co-operate with the above-mentioned nut portion.

### Problems Solved by the Invention

The objective of the present invention is to provide a double acting intramedullary bone distraction implant, which enables to carry out a shortening operation on the lengthening apparatus in cases of uncontrollable lengthening that theoretically may be guided by the patient or due to insufficient new bone formation.

Another objective of the present invention is to provide a double acting intramedullary bone distraction implant, wherein, by means of ensuring sealing via the gaskets that are used, bodily fluids are prevented from entering into the system and penetration of the substances therein to the tissues is prevented.

A further objective of the present invention is to provide a double acting intramedullary bone distraction implant wherein the risk of infections is reduced by external control and energy transmission via induction method.

Another objective of the present invention is to provide a double acting intramedullary bone distraction implant, which enables to form a security point for terminating backward movement of the system by means of forming a head member on the part which contacts the bone and moves in forward-backward direction.

### Detailed Description of the Invention

A double acting intramedullary bone distraction implant developed to fulfill the objective of the present invention is illustrated in the accompanying figures, in which:

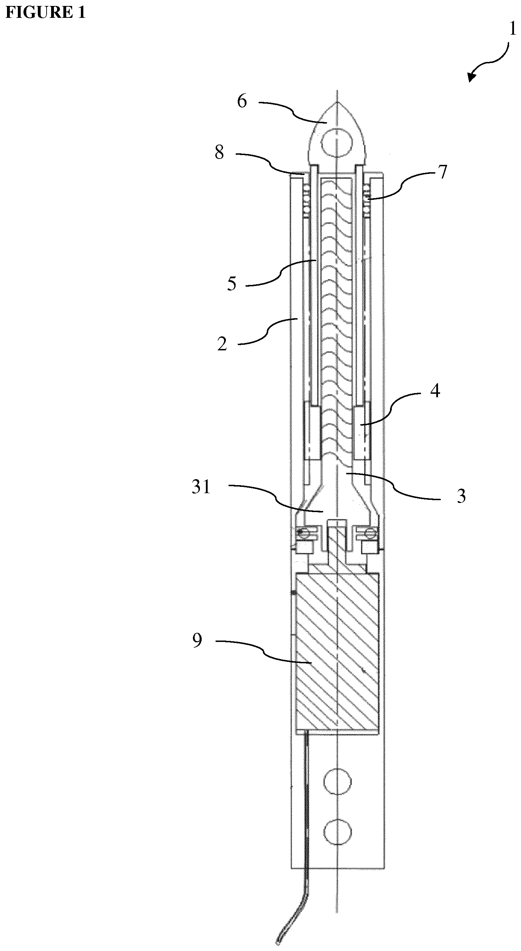

- **Figure 1**.: is a front sectional view of the double acting intramedullary bone distraction implant of the present invention.

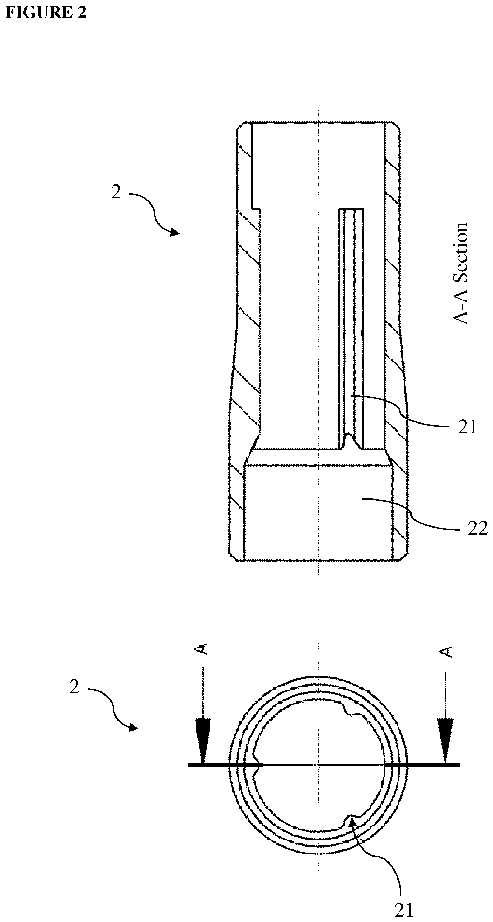

- **Figure 2.**: is a top view of the outer tube and a view of the A-A section in the double acting intramedullary bone distraction implant of the present invention.

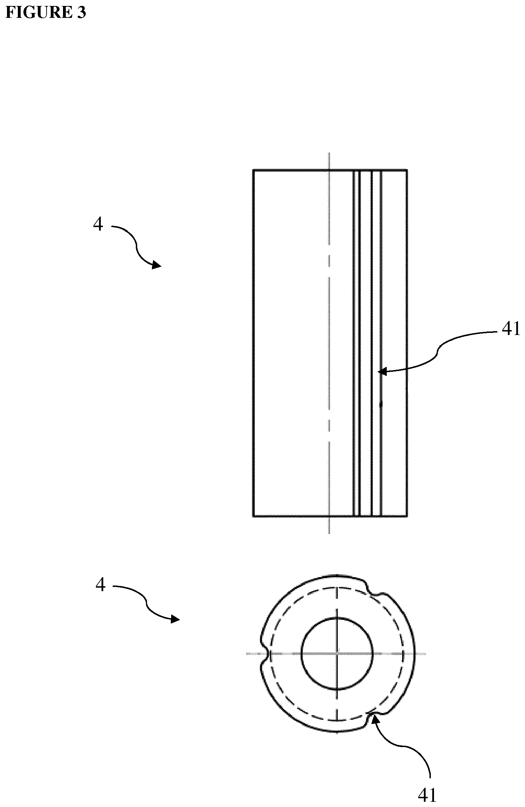

- **Figure 3.**: is a front and top view of the pusher nut in the double acting intramedullary bone distraction implant of the present invention.

The components in the figures are each given reference numbers as follows:
**1.** Bone distraction implant
**2.** Outer tube
   **21.** Guide member
   **22.** Slot
**3.** Screw
   **31.** Screw head
**4.** Pusher nut
   **41.** Canal spacing
**5.** Inner tube
**6.** Head
**7.** Gasket
**8.** Cover
**9.** Motor

A double acting intramedullary bone distraction implant (1), which can be extended or shortened in the bone in cases of uncontrolled bone growth or due to insufficient new bone formation in the patient's body, basically comprises
- at least one outer tube (2) which is in the form of a hollow rod having a size that allows fitting into the bone,
- at least one guide member (21) which is provided in the form of a protrusion on the inner space of the outer tube (2),
- at least one slot (22) which is provided in the form of a cavity on the space in the inner part of the outer tube (2),
- at least one screw (3), which is disposed into the outer tube (2) such that it can perform rotational movement around its own axis, and which includes threads on the outer surface thereof,
- at least one screw head (31), which is located on the end of the screw (3) in the form of a ridge, and which, by fitting into the slot (22), provides a security measure for preventing linear movement of the screw (3),
- at least one pusher nut (4), which is located between the screw (3) and the outer tube (2), is connected to the threads of the screw (3) by means of its threaded structure, and comprises a recess-shaped canal spacing (41) into which the guide member (21) will fit in order to prevent rotation thereof around its own axis; and which moves linearly in the outer tube (2) with the rotational movement it receives from the screw (3),
- at least one inner tube (5) which is connected to one end of the pusher nut (4) and which moves together with the pusher nut (4) in the outer tube (2) with the linear movement of the pusher nut (4),
- at least one head (6), which is secured to the inner part of the inner tube (5) such that it remains out of the outer tube (2), enables the linear movement of the inner tube (5) to be transmitted onto the bone, and is located so as to prevent fitting of the end part of the inner tube (5) into the outer tube (2),
- at least one gasket (7), which is located between the outer tube (2) and the inner tube (5), and which prevents bodily fluids from entering into the outer tube (2) and the substances therein from penetrating to the tissues,
- at least one motor (9) which is located in the outer tube (2) and which supplies rotational movement drive to the screw head (31) of the screw (3).

The bone distraction implant (1) of the present invention comprises an outer tube (2) which is placed into the bone, a motor (9) which is arranged in the outer tube (2), a screw (3) which rotates around its own axis by means of the rotation force received from the motor (9), a pusher nut (4) which is connected to the screw and which moves in linear direction with the rotation force, an inner tube (5) connected to the pusher nut (4), and a head (6) at the end of the inner tube (5). When the motor (9) transmits the rotational movement to the screw (3), the pusher rod (4) converts the received rotational movement to linear movement, and as it transmits the linear movement to the inner tube (5), the inner tube (5) moves forward and backward. Additionally, the head (6) provided at the end of the inner tube (5) in the system enables the inner tube (5) to transmit its forward and backward movement onto the bone.

The outer tube (2) provided in the bone distraction implant (1) of the present invention is preferably in the form of a hollow cylinder such that it can be placed into the bone. On one side of the outer tube (2) there is provided a motor (9) member, while on the other side thereof, there is provided an inner tube (5) which enables the bone distraction implant (1) to be extended or shortened within the bone. In the bone distraction implant (1), there is provided a screw (3) which is connected to the motor (9) and which performs a rotational movement around its own axis in the outer tube (2) by means of the drive it receives from the motor (9). The screw (3) is born on the outer tube (2) in order to prevent the screw from moving in a linear manner with the rotational movement when performing rotational movement around its own axis. There is a pusher nut (4) member between the screw (3) and the outer tube (2) in the bone distraction implant (1). The threaded structure provided on the pusher nut is located on the parts thereof that contact the screw (3). The threads of the pusher nut are compatibly located on the threads of the screw (3). Since the guide member (21) provided on the outer tube (2) engages onto the canal spacing (41) located on the pusher nut (4), the rotational movement coming from the screw (3) prevents the pusher nut (4) from rotating around its own axis. The rotational movement at the screw (3) is transmitted onto the pusher nut (4) by means of the threads provided on the screw (3) so as to allow it to perform a linear movement. By means of this configuration of the system, the pusher nut (4) can move forward or backward according to the motor (9) rotation direction in the outer tube (2). The guide member (21) on the outer tube (2) extends longitudinally along the outer tube (2) and thus enables the pusher nut (4) to perform linear movement in the outer tube (2) without performing a rotational movement. The bone distraction implant (1) of the present invention includes an inner tube (5) which is connected with the pusher nut (4) and which enables lengthening and shortening of the bone distraction implant (1) with its forward and backward movement in the outer tube (2). The inner tube is fixed onto the pusher nut (4) in the outer tube (2) such that it can move together with the pusher nut (4). The pusher nut transmits the linear movement it receives from the screw (3) onto the inner tube (5) and thus the inner tube (5) can move in linear direction within the outer tube (2). With the systems which are configured such that the movement is transmitted by the pusher nut (4); the inner tube (5), which moves in forward direction by means of the rotation force of the motor (9), can move in backward direction performing a shortening movement when the motor (9) performs a rotational movement in the opposite direction.

In order for the part of the inner tube (5) contacting the bone in the bone distraction implant (1) of the present invention to transmit the force to the bone in a suitable manner, the inner tube (5) is connected to the head (6) member at its end part. Furthermore, the head (6) forms a ridge at the part where it is connected to the inner tube (5); and when the head contacts the outer tube (2) while the inner tube (5) is being closed, it limits linear movement of the inner tube (5) and provides a security system for the closing movement of the inner tube (5).

In one embodiment of the invention, there is provided a slot (22) formed on the connection parts of the motor (9) and the screw (3) of the outer tube (2). By means of the screw head (31) provided on the screw (3) that fits in the slot (22), the screw (3) is prevented from performing a linear movement. In the case that the screw (3) tends to perform a linear movement, the screw head (31) hits the slot (22) and the bone is prevented from being injured in case of possible mechanical failures.

In one embodiment of the invention, an O-ring shaped gasket (7), which is provided for ensuring sealing between the inner tube (5) and the outer tube (2), is used. By means of this gasket (7) connection system, entrance of bodily fluids into the outer tube (2) and penetration of the substances in the outer tube (2) to the tissues are prevented.

In one embodiment of the invention, the head (6) has a conical geometry. Thanks to the conical structure of the head (6) member, the pulling force that may act on the head (6) is enabled to be conveniently distributed on the head (6) and the mechanical strength on the end of the thread on the inner tube (5) is enabled to be enhanced.

In one embodiment of the invention, an external control unit is provided for control of the motor (9) located in the outer tube (2). By means of the control unit and the electric transmitter system provided out of the body, the energy and the control signals are delivered into the body; and the signals are collected by a receiver that will be placed into the body and are transmitted to the bone distraction implant (1).

In one embodiment of the invention, the power supply required for operation of the motor (9) member is transmitted to the motor (9) member via induction method. The patient is prevented from getting infected by means of the energy transmission via induction method.

In one embodiment of the invention, there is provided a cover (8) member located at the end part of the outer tube (2) so as to cover the top of the inner part of the outer tube (2). The cover (8) member prevents entrance of substances into the outer tube (2) from the outer environment, and prevents deformation of the outer tube (2) when the head (6) member hits thereon.

## Claims

1. A double acting intramedullary bone distraction implant (1), which can be extended or shortened in the bone in cases of uncontrolled bone growth or due to insufficient new bone formation in the patient's body, and **comprising;**
- at least one outer tube (2) which is in the form of a hollow rod having a size that allows fitting into the bone,
- at least one guide member (21) which is provided in the form of a protrusion on the inner space of the outer tube (2),
- at least one screw (3), which is disposed into the outer tube (2) such that it can perform rotational movement around its own axis, and which includes threads on the outer surface thereof,
- at least one pusher nut (4), which is located between the screw (3) and the outer tube (2), is connected to the threads of the screw (3) by means of its threaded structure, and comprises a recess-shaped canal spacing (41) into which the guide member (21) will fit in order to prevent rotation thereof around its own axis; and which moves linearly in the outer tube (2) with the rotational movement it receives from the screw (3),
- at least one inner tube (5) which is connected to one end of the pusher nut (4) and which moves together with the pusher nut (4) in the outer tube (2) with the linear movement of the pusher nut (4),
- at least one motor (9) which is located in the outer tube (2) and which supplies rotational movement drive to the screw head (31) of the screw (3) and **characterized by**
- at least one head (6) which is secured to the inner part of the inner tube (5) such that it remains out of the outer tube (2), enables the linear movement of the inner tube (5) to be transmitted onto the bone, and is located so as to prevent fitting of the end part of the inner tube (5) into the outer tube (2).

2. A bone distraction implant (1) according to Claim 1, **characterized by** at least one slot (22) which is provided in the form of a cavity on the space in the inner part of the outer tube (2).

3. A bone distraction implant (1) according to Claim 2, **characterized by** at least one screw head (31), which is located on the end of the screw (3) in the form of a ridge, and which, by fitting into the slot (22), provides a security measure for preventing linear movement of the screw (3).

4. A bone distraction implant (1) according to Claim 1, **characterized by** at least one gasket (7), which is located between the outer tube (2) and the inner tube (5), and which prevents bodily fluids from entering into the outer tube (2) and the substances therein from penetrating to the tissues.

5. A bone distraction implant (1) according to Claim 2, **characterized by** the slot (22), which prevents the pulling forces that may be applied on the head (6) from acting on the screw (3) so as to displace the screw (3) in axial direction, and which enables the tension that may be formed at the thread end region to be distributed on the conical surface.

6. A bone distraction implant (1) according to Claim 1, **characterized by** at least one control unit which enables to control the motor (9) in the outer tube (2) by having the control unit and the electric transmitter system provided out of the body deliver the energy and the control signals into the body; and having the signals collected by a receiver that will be placed into the body.

7. A bone distraction implant (1) according to any one of the preceding claims, **characterized in that** the power supply required for operation of the motor (9) member is transmitted via induction method.

8. A bone distraction implant (1) according to Claim 1, **characterized by** at least one cover (8), which is located at the end part of the outer tube (2) so as to cover the top of the inner part of the outer tube (2), and which prevents entrance of substances into the outer tube (2).

## Patentansprüche

1. Doppelwirkendes intramedulläres Knochendistraktionsimplantat (1), das im Knochen bei unkontrolliertem Knochenwachstum oder aufgrund unzureichender Knochenneubildung im Körper des Patienten verlängert oder verkürzt werden kann, und **umfassend;**
- mindestens ein Außenrohr (2), das in Form einer Hohlstange mit einer Größe vorliegt, die ein Einpassen in den Knochen ermöglicht,
- mindestens ein Führungselement (21), das in Form eines Vorsprungs im Innenraum des Außenrohrs (2) vorgesehen ist,
- mindestens eine Schraube (3), die in dem Außenrohr (2) so angeordnet ist, dass sie eine Drehbewegung um ihre eigene Achse ausführen kann, und die Gewinde auf ihrer Außenfläche enthält,
- mindestens eine Druckmutter (4), die sich zwischen der Schraube (3) und dem Außenrohr (2) befindet, mit den Gewinden der Schraube (3) über ihre Gewindestruktur verbunden ist und einen vertiefungsförmigen Kanalabstand (41) aufweist, in den das Führungselement (21) passt, um dessen Drehung um seine eigene Achse zu verhindern; und die sich linear im Außenrohr (2) mit der von der Schraube (3) aufgenommenen Drehbewegung bewegt,
- mindestens ein Innenrohr (5), das mit einem Ende der Druckmutter (4) verbunden ist und sich mit der Druckmutter (4) im Außenrohr (2) mit der Linearbewegung der Druckmutter (4) bewegt,
- mindestens einen Motor (9), der sich im Außenrohr (2) befindet und dem Schraubenkopf (31) der Schraube (3) einen Drehbewegungsantrieb zuführt.
und **gekennzeichnet durch**
- mindestens einen Kopf (6), der am inneren Teil des Innenrohrs (5) so befestigt ist, dass er aus dem Außenrohr (2) heraus bleibt, die lineare Bewegung des Innenrohrs (5) auf den Knochen überträgt und so angeordnet ist, dass ein Einpassen des Endteils des Innenrohrs (5) in das Außenrohr (2) verhindert wird.

2. Knochendistraktionsimplantat (1) nach Anspruch 1, **gekennzeichnet durch** mindestens einen Schlitz (22), der in Form eines Hohlraums auf dem Raum im Innenteil des Außenrohrs (2) vorgesehen ist.

3. Knochendistraktionsimplantat (1) nach Anspruch 2, **gekennzeichnet durch** mindestens einen Schraubenkopf (31), der sich am Ende der Schraube (3) in Form eines Grates befindet und der durch Einpassen in den Schlitz (22) eine Sicherheitsmaßnahme zum Verhindern einer linearen Bewegung der Schraube (3) bietet.

4. Knochendistraktionsimplantat (1) nach Anspruch 1, **gekennzeichnet durch** mindestens eine Dichtung (7), die sich zwischen dem Außenrohr (2) und dem Innenrohr (5) befindet und verhindert, dass Körperflüssigkeiten in das Außenrohr (2) gelangen und die darin enthaltenen Substanzen in das Gewebe eindringen.

5. Knochendistraktionsimplantat (1) nach Anspruch 2, **gekennzeichnet durch** den Schlitz (22), der verhindert, dass die auf den Kopf (6) aufbringbare Zugkräfte auf die Schraube (3) einwirken, um die Schraube (3) in axialer Richtung zu verschieben, und der es ermöglicht, dass die im Gewindesendbereich gebildete Spannung auf die konische Oberfläche verteilt wird.

6. Knochendistraktionsimplantat (1) nach Anspruch 1, **gekennzeichnet durch** mindestens eine Steuereinheit, die es ermöglicht, den Motor (9) im Außenrohr (2) zu steuern, indem die Steuereinheit und das aus dem Körper vorgesehene elektrische Sendersystem die Energie und die Steuersignale in den Körper liefern; und die Signale von einem Empfänger gesammelt werden, der in den Körper eingesetzt wird.

7. Knochendistraktionsimplantat (1) nach einem der vorhergehenden Ansprüche, **dadurch gekennzeichnet, dass** die für den Betrieb des Motor (9) Elements erforderliche Energieversorgung über ein Induktionsverfahren übertragen wird.

8. Knochendistraktionsimplantat (1) nach Anspruch 1, **gekennzeichnet durch** mindestens eine Abdeckung (8), die am Endteil des Außenrohrs (2) so angebracht ist, dass sie die Oberseite des Innenteils des Außenrohrs (2) abdeckt und den Eintritt von Substanzen in das Außenrohr (2) verhindert.

## Revendications

1. Implant de distraction osseuse intramédullaire à double action (1), qui peut être allongé ou raccourci dans l'os en cas de croissance osseuse incontrôlée ou en raison d'une formation osseuse nouvelle insuffisante dans le corps du patient, et **comprenant ;**
- au moins un tube extérieur (2) qui se présente sous la forme d'une tige creuse dont la taille permet l'insertion dans l'os,
- au moins un élément de guidage (21) qui est prévu sous la forme d'une saillie sur l'espace intérieur du tube extérieur (2),
- au moins une vis (3) qui est disposée dans le tube extérieur (2) de manière à pouvoir effectuer un mouvement de rotation autour de son propre axe, et qui comporte des filets sur sa surface extérieure,
- au moins un écrou de poussoir (4) situé entre la vis (3) et le tube extérieur (2) est relié aux filets de la vis (3) au moyen de sa structure filetée, et comprend un espacement de canal (41) en forme de creux dans lequel l'élément de guidage (21) va s'insérer pour empêcher sa rotation autour de son propre axe ; et qui se déplace linéairement dans le tube extérieur (2) avec le mouvement de rotation qu'il reçoit de la vis (3),
- au moins un tube intérieur (5) qui est relié à une extrémité de l'écrou de poussoir (4) et qui se déplace avec l'écrou de poussoir (4) dans le tube extérieur (2) avec le déplacement linéaire de l'écrou de poussoir (4),
- au moins un moteur (9) qui se trouve dans le tube extérieur (2) et qui alimente la tête de vis (31) de la vis (3) en mouvement de rotation et qui est **caractérisé par**
- au moins une tête (6) qui est fixée à la partie intérieure du tube intérieur (5) de manière à rester hors du tube extérieur (2), permet de transmettre le mouvement linéaire du tube intérieur (5) sur l'os, et est situé de manière à empêcher la fixation de l'extrémité du tube intérieur (5) dans le tube extérieur (2).

2. Implant de distraction osseuse (1) selon la revendication 1, **caractérisé par** au moins une fente (22) qui est prévue sous la forme d'une cavité sur l'espace dans la partie intérieure du tube extérieur (2).

3. Implant de distraction osseuse (1) selon la revendication 2, **caractérisé par** au moins une tête de vis (31), qui est située à l'extrémité de la vis (3) sous la forme d'une nervure, et qui, en s'adaptant dans la fente (22), fournit une mesure de sécurité pour empêcher un mouvement linéaire de la vis (3).

4. Implant de distraction osseuse (1) selon la revendication 1, **caractérisé par** au moins un joint (7), qui est situé entre le tube extérieur (2) et le tube intérieur (5), et qui empêche les fluides corporels d'entrer dans le tube extérieur (2) et les substances qui s'y trouvent de pénétrer dans les tissus.

5. Implant de distraction osseuse (1) selon la revendication 2, **caractérisé par** la fente (22), qui empêche les forces de traction qui peuvent être appliquées sur la tête (6) d'agir sur la vis (3) de manière à déplacer la vis (3) en direction axiale, et qui permet de répartir sur la surface conique la tension qui peut se former au niveau de l'extrémité du fil.

6. Implant de distraction osseuse (1) selon la revendication 1, **caractérisé par** au moins une unité de commande qui permet de commander le moteur (9) dans le tube extérieur (2) en ayant l'unité de commande et le système d'émetteur électrique prévu à partir du corps fournissent l'énergie et les signaux de commande dans le corps ; et ayant les signaux recueillis par un récepteur qui sera placé dans le corps.

7. Implant de distraction osseuse (1) selon l'une quelconque des revendications précédentes, **caractérisé en ce que** l'alimentation électrique nécessaire au fonctionnement de l'élément moteur (9) est transmise par un procédé à induction.

8. Implant de distraction osseuse (1) selon la revendication 1, **caractérisé par** au moins un couvercle (8), qui est situé à la partie d'extrémité du tube extérieur (2) de manière à recouvrir la partie supérieure de la partie intérieure du tube extérieur (2), et qui empêche l'entrée de substances dans le tube extérieur (2).
